Europäisches Patentamt

European Patent Office

Office européen des brevets

(11) Publication number: **0 405 403 A2**

# EUROPEAN PATENT APPLICATION

(21) Application number: 90112042.8

(22) Date of filing: 25.06.90

(51) Int. Cl.5: **A61F 13/80**

(30) Priority: 29.06.89 DE 3921385

(43) Date of publication of application:
02.01.91 Bulletin 91/01

(84) Designated Contracting States:
AT BE CH DE DK ES FR GB GR IT LI LU NL SE

(71) Applicant: THE PROCTER & GAMBLE
COMPANYANY
One Procter & Gamble Plazaaza
Cincinnati Ohio 45202(US)

(72) Inventor: Haubach, Claus C.
Defregger Strasse 1
D-6700 Ludwigshafen(DE)
Inventor: Beisel-Haubach, Margarita
Defregger Strasse 1
D-6700 Ludwigshafen(DE)

(74) Representative: Patentanwälte TER MEER -
MÜLLER - STEINMEISTER & PARTNERNER
Mauerkircherstrasse 45
D-8000 München 80(DE)

(54) Liners for ladies' panties.

(57) The invention relates to a liner for ladies' panties, consisting of a fluid-impermeable lower ply (1),
an absorbent middle ply (2) and a fluid-permeable
upper ply (3). According to the invention, the liner
exhibits at least one longitudinal fold (7) which allows
an adaptation of the width of the insert to the width
of the crotch part (6) of the ladies' panties. The
longitudinal fold (7) can be arranged in the central
longitudinal axis and, preferably, is releasably closed
by a sticking bond at least in the central part (9).

Fig. 1

EP 0 405 403 A2

# LINERS FOR LADIES' PANTIES

The invention relates to a liner for ladies panties, consisting of a fluid-impermeable lower ply, an absorbent middle ply and a fluid-permeable upper ply.

Such known liners are used both during menstruation and in daily use. Even though there is a large number of sizes of ladies' panties, these liners are as a rule produced by every manufacturer in only one size or at best in two sizes, so that these liners are, with respect to their width, either wider than the crotch part, so that they project out of the panties and cause discomfort to the wearer, or are smaller than the crotch part, which reduces the area of optimum protective action and involves the risk of soiling the part of the panties, which is not covered, in particular during menstruation. On the free side of the lower ply, such liners have covered adhesive strips or sticking strips, which serve as fixing means. For use, the cover layers are removed from the adhesive layer or sticking layer within the ladies' panties, so that firm seating of the liner, without a possibility of sliding, is ensured.

It is the object of the invention to provide a liner for ladies' panties, which allows optimum utilisation of the particular width of the crotch part of the ladies' panties, without the liner projecting laterally from the ladies' panties or not fully covering the crotch part thereof.

This object is achieved by a liner having the features of Claim 1.

Advantageous embodiments of the invention are to be found in the subclaims.

The invention is explained below by means of illustrative embodiments with reference to drawings in which, in diagrammatic illustration:

Figure 1 shows a liner with a closed longitudinal fold and a transversely arranged closure,

Figure 2 shows a liner with a closed longitudinal fold with a closure arranged in the central part,

Figure 3 shows a cross-section, not true to scale, along the line III-III in Figure 1,

Figure 4 shows a liner with lateral tabs and a partially open longitudinal fold in the arrangement in the crotch part of ladies' panties, and

Figure 5 shows the same as Figure 4, but with the lateral tabs closed around the crotch part.

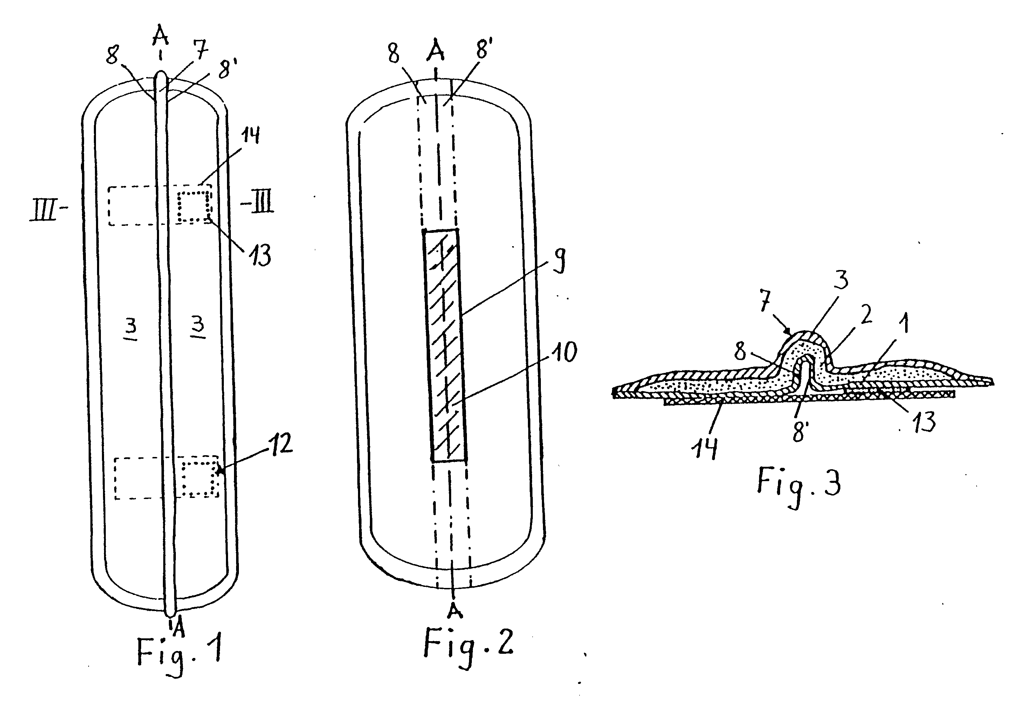

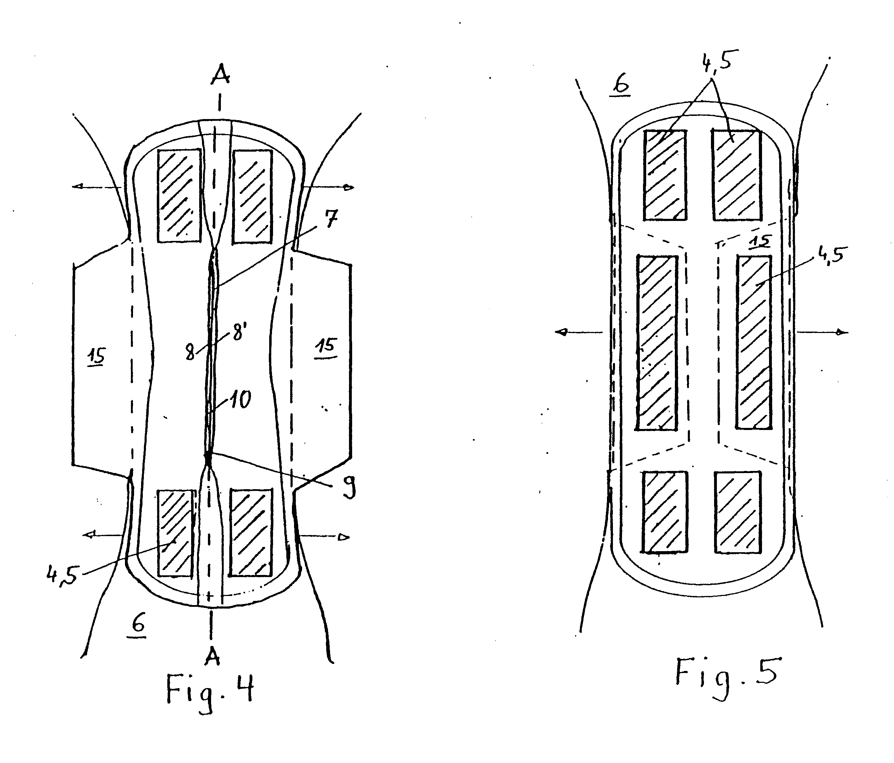

Each liner has a fluid-impermeable lower ply 1, an absorbent middle ply 2 arranged on top thereof and a fluid-permeable upper ply 3 (Figure 3). The liner has an elongate shape with +- parallel side edges in mirror symmetry and is provided with approximately semi circular ends. On the free side of the lower ply 1, removable cover strips 4 for covered sticking strips 5 are usually provided, by means of which the liner can, after removal of the cover strips 4, be stuck in a fixed position into the crotch part 6 (Figures 4 and 5) of ladies' panties. Such liners are widely known and are therefore also not explained in more detail with respect to the material of the individual plies and their joining.

Each liner has at least one longitudinal fold 7, which is preferably made in the longitudinal axis of symmetry A-A of the insert. This central arrangement of the longitudinal fold 7 is preferred, since it corresponds to the shape of the body, whereas a possible off-centre arrangement might, at least if only a single main longitudinal fold 7 were provided, lead to discomfort in sitting and to the formation of a pressure line on the body.

In order to enable the width of the liner to be adapted to the width of any given crotch part 6 of the ladies' panties, the longitudinal fold 7 is releasably closed.

According to an illustrative embodiment, the sides 8, 8' (Figure 2) of the longitudinal fold 7 are provided in the central part 9 of the longitudinal fold 7 with an adhesive bond or sticking bond 10, which can consist, for example, of separable sticking layers, which can have been applied to the lower ply 1, for example in the form of a closed strip 11 of adhesive for both sides 8, 8', or in the form of separate sticking strips for each side 8 and 8'.

The provision of the adhesive bond or sticking bond 10 only in the central part 9 (Figures 2 and 4) is sufficient, since the ends of the liner are already located in the wider transition part from the crotch part 6 into the actual panty and sufficient width is available. Moreover, a closed longitudinal fold 7 in the region of the ends - which is also possible in principle - might ride up slightly and lead to discomfort.

It is also possible, however, to provide, transversely to the longitudinal fold 7, adjustable closures 12 (Figure 1) which can consist, for example, of an adhesive spot 13 or strip, arranged on one side 8' of the longitudinal fold 7, and an adhesive strip 14 which is arranged on the opposite side 8' of the longitudinal fold 7 and serves for fixing once or repeatedly to the adhesive spot 13.

Depending on the adjustment of the releasable bonds or corresponding to the pulling-apart or the degree of opening of the longitudinal fold 7, that is to say according to the extent to which the two sides 8, 8' of the old longitudinal fold 7 are pulled apart in the region of their adhesive bond perpendicular to the longitudinal extent of the liner (see the arrows in Figures 4 and 5) or corresponding to the lengthening of the adhesive strips 14 of the closures 12 (see Figure 3), the width of the liner can be continuously adjusted until the longitudinal fold(s) 7 etc. (Figure 2) are fully open, so that adaptation to virtually any desired width of the crotch part 6 of ladies' panties is possible, whereby in turn optimum utilisation of the liner and hence also optimum protection is achieved, without causing discomfort to the wearer. If the longitudinal fold 7 is not completely released, it conveys a feeling of safety to the wearer, but without hindering her.

In order to make greater width extension possible, additional further parallel longitudinal folds (not shown) can be provided if necessary. However, these should then advantageously be made smaller, in order to prevent riding up or possible pressure marks.

It is possible and advantageous to provide the liners, in particular those which are to be worn during menstruation, with lateral tabs 15 (Figure 5), which can represent lateral extensions of the lower ply and-or the upper ply 3. The lateral tabs 15 have known adhesive strips or sticking strips 5, by means of which they are fixed, when they are turned over outwards onto the outer crotch part 6 (Figure 5), either directly - at least partially - to the crotch part 6 of the ladies' panties or to one another, so that the liner is thus provided with an additional firmer seating, the crotch part 6 being almost completely enclosed by the lateral tabs 15 right at the top.

As the adhesive bond or sticking bond 10 or as covered adhesive strips 5, any desired suitable adhesives, two-sided adhesive strips or also so-called touch-and-close closures can be used, which still ensure a firm bond even when they are partially open.

## Claims

1. Liner for ladies' panties, consisting of a fluid-impermeable lower ply (1), an absorbent middle ply (2) and a fluid-permeable upper ply (3), characterized in that it exhibits at least one longitudinal fold (7) which allows an adaptation of the width of the liner to the width of the crotch part (6) of the ladies' panties.

2. Liner according to Claim 1, characterized in that a longitudinal fold (7) is arranged in the central longitudinal axis.

3. Liner according to Claim 1 or Claim 2, characterized in that each longitudinal fold (7) is releasably closed at least in the central part (9).

4. Liner according to one of Claims 1 to 3, characterized in that each longitudinal fold (7) is at least partially closed by means of adhesive bonds and/or sticking bonds (10).

5. Liner according to Claim 4, characterized in that the adhesive bond and/or sticking bond (10) in the longitudinal fold (7) is arranged to join the two sides (8, 8') of the longitudinal fold (7) to one another.

6. Liner according to Claim 4, characterized in that the adhesive bonds and/or sticking bonds (10) are arranged transversely to the longitudinal fold.

7. Liner according to one of Claims 1 to 6, characterized in that the liner has, on both sides, lateral tabs (15) with sticking bonds for additional fixing of the lateral tabs (15) to the outside of the crotch part (6) of the ladies' panties.

Fig.1

Fig.2

Fig.3

Fig. 5

Fig. 4